# EUROPEAN PATENT APPLICATION

(11) **EP 4 410 819 A1**
(43) Date of publication of application: **07.08.2024**
(21) Application number: 22876781.0
(22) Date of filing: 27.09.2022
(51) Int. Cl.: C07K 7/08, A61K 38/00, A61P 29/00, A61K 8/64, A61Q 19/00

(54) **PEPTIDE HAVING ANTI-INFLAMMATORY ACTIVITY AND USE THEREOF**

(30) Priority: 30.09.2021 KR 20210130420
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Heinonen & Co
(86) International application number: PCT/KR2022/014394
(87) International publication number: WO 2023/055009

(57) **Abstract**

The present invention relates to a peptide having anti-inflammatory activity and an anti-inflammatory composition comprising same. A peptide comprising the amino acid sequence of SEQ ID NO: 1, according to the present invention has anti-inflammatory activity, and thus can be used as a raw material of medicines for the prevention, amelioration or treatment of inflammatory diseases or cosmetics for preventing or ameliorating skin inflammation.

## Description

### TECHNICAL FIELD

The present invention relates to a peptide having anti-inflammatory activity and an anti-inflammatory composition and an anti-inflammatory composition including the same as an active ingredient.

### BACKGROUND ART

Inflammation, being one of defense responses of living tissue against external stimuli, is a mechanism to repair and regenerate damage caused by physical action or harmful substances, chemical stimulation, bacterial infection, and sustained inflammatory responses rather promote mucosal damage, which in some cases causes various diseases, including cancer. Lipopolysaccharide (LPS), which is an outer membrane component of Gram-negative bacteria, causes various reactions such as local inflammation, antibody production, and sepsis, and macrophages respond in the early stage of LPS infection and play a central role in host defense and homeostasis. However, high concentration LPS stimulation causes macrophages to secrete pro-inflammatory mediators, such as tumor necrosis factor-a (TNF-α), interleukin (IL)-1 and IL-6, and nitric oxide (NO), thereby resulting in fatal outcomes for the host. Generally, NO is produced from inducible NO synthase (iNOS) when macrophages are activated and has an antibacterial effect that inhibits some viruses and parasites; however, excessive NO formation is known to cause inflammation and cause tissue damage, genetic mutation, nerve damage, etc.

The expression of pro-inflammatory cytokines, such as TNF-α, IL-1, and IL-6, is regulated by mitogen-activated protein kinases (MAPKs), such as extracellular signal-regulated kinase1/2(ERK1/2), p38 kinases (p38), and c-Jun NH2-terminal kinase (JNK), and nuclear factor kappa B (NF-κB). NF-κB plays an important role in the expression of genes relating to immunity and inflammatory responses, and when NF-κB is activated, inhibitory kappa B α (IκBα) bound to NF-κB is decomposed and NF-κB enters the nucleus from the cell cytoplasm, and then acts as a transcription factor for the expression of cytokines, such as TNF-α, IL-12, and IL-6.

Meanwhile, interleukin-17 (IL-17) is known to be closely associated with chronic inflammation. IL-17 is an inflammatory cytokine that plays an important role in the host's defense mechanisms against extracellular bacterial and fungal infections, and in particular, T-helper 17 (Th17) cells play an important role in the production and differentiation of IL-17. The increase of production and secretion of IL-17 *in vivo* is closely associated with various inflammatory and autoimmune diseases, including psoriasis, psoriatic arthritis (PsA), rheumatoid arthritis (RA), atopic dermatitis (AD), acne lesions, and ankylosing spondylitis (AS). With the identification of the major pathogenic roles of IL-17 and Th17 cells in inflammatory and autoimmune diseases, their inhibition and regulation have been proposed as important strategies for treating diseases, and potential target treatment methods, etc. through inhibition and neutralization of Th17 cell line cytokines and inhibition and regulation of specific transcription factors are being studied.

Non-steroidal anti-inflammatory drugs (NSAIDS), which are currently used as anti-inflammatory drugs, exhibit anti-inflammatory effects by inhibiting the activity of the enzyme called cyclooxygenase (COX) involved in the biosynthesis of prostaglandin from arachidonic acid; however, they cause serious side effects, such as gastrointestinal disorders, liver disorders, and renal disorders, in addition to the main therapeutic effects, and thus there are limitations on long-term use of these drugs.

Accordingly, there is a need for the development of next-generation anti-inflammatory drugs that can overcome the limitations of drugs that act on the central nervous system and the gastrointestinal tract. Recently, the importance in relation to the direction of development of anti-inflammatory drugs has been expanded (non-patent literature) by way of shifting from drugs acting on the central nervous system and the gastrointestinal tract to developing drugs with local action, and from small molecule synthetic compounds for oral administration to the development of synthetic peptides for subcutaneous or intravascular injection. The development of synthetic peptide therapeutics is a promising driving force in the future biopharmaceutical industry, as it has great potential for clinical effectiveness in the field of development of anti-inflammatory drugs, discovery of new drug targets and mechanisms, development of peptide-based drugs, and development of delivery optimization technologies are very important.

### [Prior Art Document]

### [Non-Patent Document]

(Non-Patent Document 1) Sara La Manna et al., Peptides as Therapeutic Agents for Inflammatory-Related Diseases. Int. J. Mol. Sci. 2018; 19: 2714

### DISCLOSURE OF THE INVENTION

### TECHNICAL PROBLEM

An object of the present invention is to provide a peptide having anti-inflammatory activity.

Another object of the present invention is to provide an anti-inflammatory composition including the peptide having the above-described activity as an active ingredient.

Still another object of the present invention is to provide a composition for preventing or treating an inflammatory disease including the peptide having the above-mentioned activity as an active ingredient.

Still another object of the present invention is to provide a cosmetic composition for anti-inflammation including the peptide having the above-mentioned activity as an active ingredient.

### TECHNICAL SOLUTION

In order to achieve the above objects, an aspect of the present invention provides a peptide including the amino acid sequence represented by SEQ ID NO: 1.

Another aspect of the present invention provides an anti-inflammatory composition including the peptide as an active ingredient.

Still another aspect of the present invention provides a pharmaceutical composition for preventing or treating an inflammatory disease including the peptide as an active ingredient.

Still another aspect of the present invention provides a cosmetic composition for anti-inflammation including the peptide as an active ingredient.

### ADVANTAGEOUS EFFECTS

The peptide including an amino acid sequence of SEQ ID NO: 1 according to the present invention has anti-inflammatory activity, and thus can be used as a raw material of a pharmaceutical drug or cosmetics for the purpose of preventing, ameliorating, or treating inflammatory diseases.

The effects of the present invention are not limited to the effects mentioned above, and other effects not mentioned will be clearly understood by those skilled in the art from the following description.

### BRIEF DESCRIPTION OF THE DRAWINGS

FIG. 1 is an image of RT-PCR electrophoresis showing the effect of the peptide of the present invention on the expression of COX-2 and IFN-γ induced by LPS in splenocytes.
FIG. 2A is a graph of ELISA results showing the effect of the peptide of the present invention on the secretion of TNF-α induced by LPS in splenocytes.
FIG. 2B is a graph of ELISA results showing the effect of the peptide of the present invention on the secretion of IFN-γ induced by LPS in splenocytes.
FIG. 2C is a graph of ELISA results showing the effect of the peptide of the present invention on the secretion of IL-17 induced by LPS in splenocytes.
FIG. 3A is a graph of ELISA results showing the effect of the peptide of the present invention on the secretion of TNF-α in splenocytes.

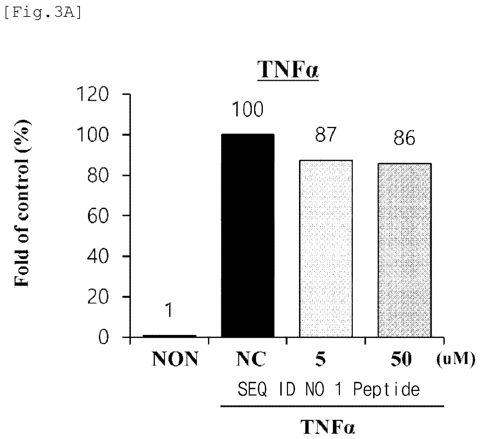

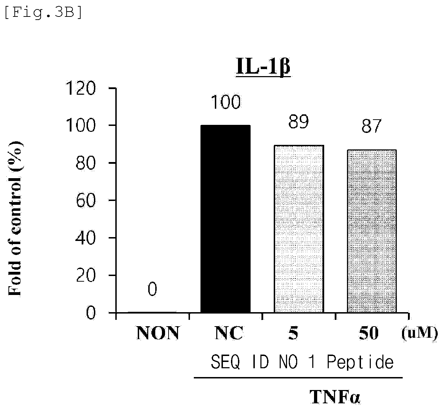

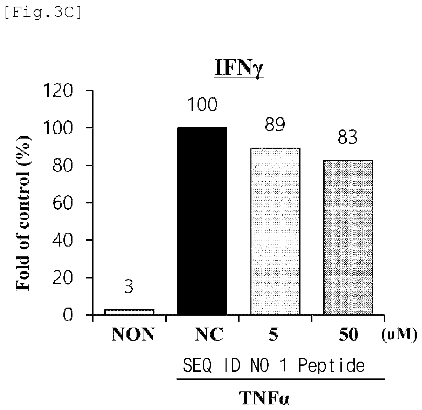

FIG. 3Bis a graph of ELISA results showing the effect of the peptide of the present invention on the secretion of IL-1β induced by TNF-α in splenocytes.
FIG. 3C is a graph of ELISA results showing the effect of the peptide of the present invention on the secretion of IFN-γ induced by TNF-α in splenocytes.

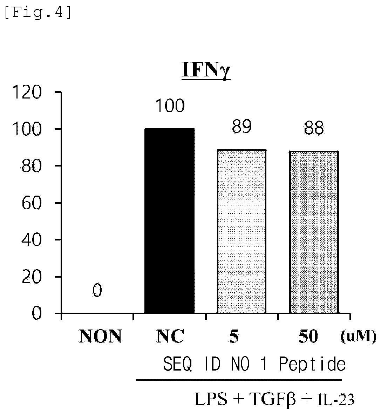

FIG. 4 is a graph of ELISA results showing the effect of the peptide of the present invention on the secretion of IFN-γ induced by LPS, TGF-β, and IL-23 in splenocytes.

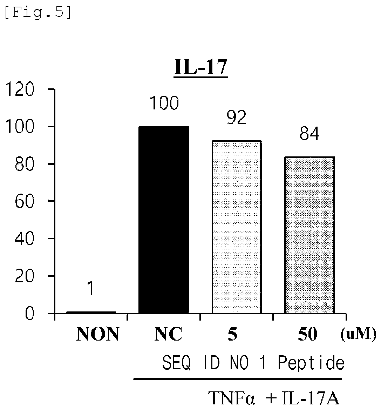

FIG. 5 is a graph of ELISA results showing the effect of the peptide of the present invention on the secretion of IL-17 induced by TNF-α and IL-17A in splenocytes.

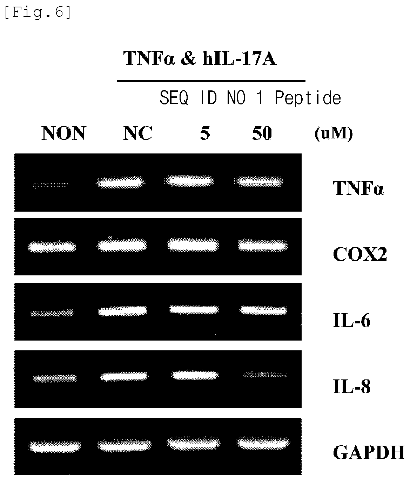

FIG. 6 is an image of RT-PCR electrophoresis showing the effect of the peptide of the present invention on the expression of TNF-α, COX2, IL-6, and IL-8 induced by TNF-α and hIL-17A in keratinocytes.

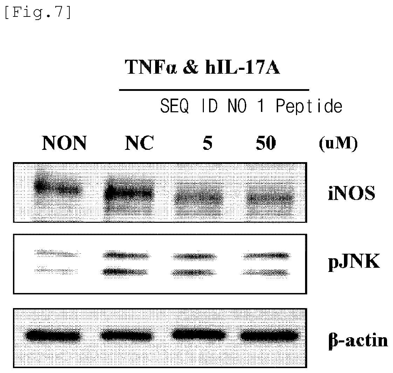

FIG. 7 is an image of Western blot electrophoresis showing the effect of the peptide of the present invention on the expression of iNOS and JNK induced by TNF-α and hIL-17A in keratinocytes.

### MODE FOR CARRYING OUT THE INVENTION

Hereinafter, the present invention will be described in detail.

An aspect of the present invention provides a peptide including the amino acid sequence represented by SEQ ID NO: 1.

As used herein, the term "peptide" refers to a linear molecule consisting of amino acid residues, and specifically, the peptide of the present invention may include the amino acid sequence represented by SEQ ID NO: 1.

The "peptide" may be variants or fragments of amino acids having different sequences due to deletion, insertion, substitution, or combinations of amino acid residues, to the extent that they do not affect its function. Amino acid exchanges that do not change the entire activity of the peptide are known in the art. In some cases, the peptide may be modified by phosphorylation, sulfation, acrylation, glycosylation, methylation, farnesylation, etc. Accordingly, the peptide of the present invention includes a peptide having an amino acid sequence substantially identical to the peptide containing the amino acid sequence of SEQ ID NO: 1, and a variant or active fragment thereof.

The substantially identical protein refers to an amino acid sequence having sequence homology of 75% or more, preferably 80% or more, for example, 85% or more, 90% or more, 95% or more, 98% or more, or 99% or more with the amino acid sequence of SEQ ID NO: 1, but is not limited thereto, and as long as the protein has 75% or more of amino acid sequence homology and has the same activity, it is included in the scope of the present invention.

In addition, the peptide of the present invention may additionally include a targeting sequence, a tag, a labeled residue, an amino acid sequence prepared for the specific purpose of increasing half-life or stability of the peptide.

Additionally, in order to obtain better chemical stability, enhanced pharmacological properties (half-life, absorption, titer, efficacy, etc.), altered specificity (e.g., a broad spectrum of biological activity), and reduced antigenicity, a protecting group may be attached to the N-terminus or C-terminus of the peptide of the present invention. The term "stability" is used to include not only *in vivo* stability, which protects the peptide of the present invention from attack by proteases *in vivo,* but also storage stability (e.g., storage stability at room temperature).

The N-terminal modification may be one in which a protecting group selected from the group consisting of an acetyl group, a fluoreonylmethoxycarbonyl group, a formyl group, a palmitoyl group, a myristyl group, a stearyl group, and polyethylene glycol (PEG) is bound to the N-terminus of the peptide, but is not limited thereto.

The C-terminal modification may be one in which a hydroxyl group (-OH), an amino group (-NH₂), azide (-NHNH₂), etc. is bound to the C-terminus of the peptide, but is not limited thereto.

Synthesis of the peptide of the present invention may be performed, for example, using a device or genetic engineering techniques. When the synthesis is performed using a device, a desired peptide may be synthesized using the Fmoc solid-phase method in an automatic peptide synthesizer.

In a specific example of the present invention, the peptide including the amino acid sequence of SEQ ID NO: 1 of the present invention was synthesized and identified using the Fmoc solid-phase method, and the identified peptide was selected by verifying its efficacy.

In a specific example of the present invention, in order to verify the efficacy of the identified peptide, the peptide of the present invention was selected through validation of the effect of inhibiting the expression or activity of inflammatory cytokines.

Accordingly, the peptide including the amino acid sequence of SEQ ID NO: 1 of the present invention has anti-inflammatory activity.

Another aspect of the present invention provides an anti-inflammatory composition including a peptide including the amino acid sequence of SEQ ID NO: 1 as an active ingredient.

The term "anti-inflammatory" or "inhibition of inflammation" is used as a meaning to include amelioration (alleviation of symptoms), treatment, and inhibition or delay of the occurrence of an inflammatory disease, as defined below.

In a specific embodiment of the present invention, the peptide including the amino acid sequence of SEQ ID NO: 1 of the present invention was shown to inhibit the expression of cyclooxygenase-2 (COX-2) and interferon-gamma (IFN-γ) in splenocytes stimulated with LPS, and to inhibit the secretion of any one inflammatory cytokine selected from the group consisting of tumor necrosis factor alpha (TNF-α), INF-γ, and interleukin 17 (IL-17).

In addition, the peptide was shown to inhibit the secretion of any one inflammatory cytokine selected from the group consisting of TNF-α, interleukin-1β (IL-1β), and IFN-γ in splenocytes stimulated with TNF-α.

In addition, it was found that the peptide inhibits the secretion of IFN-γ in splenocytes stimulated by LPS, TGF-β, and IL-23, and inhibits the secretion of IL-17 in splenocytes stimulated by TNF-α and IL-17A.

In addition, it was found that the peptide inhibits the expression of inflammatory cytokines (e.g., TNF-α, IL-6, and IL-8) and COX2 in HaCaT cells, which is an *in vitro* test model for psoriasis dermatitis induced by TNF-α and IL-17A, and reduces the activity of inflammatory mediators (e.g., iNOS and JNK).

It can be seen from these results that the peptide of the present invention has an anti-inflammatory effect by inhibiting inflammatory cytokines through regulating the expression of inflammatory cytokine genes and blocking inflammatory reactions, such as by, and inhibiting productions of PGE2 and NO and inhibiting JNK activity through regulating the expressions of COX-2 and INOS. In addition, these results indicate that the peptide of the present invention not only has a therapeutic effect on general inflammatory diseases through its existing anti-inflammatory activity, but also has an effect on treating autoimmune diseases including psoriasis.

Still another aspect of the present invention provides a pharmaceutical composition for preventing or treating inflammatory diseases including a peptide including the amino acid sequence of SEQ ID NO: 1 as an active ingredient.

As used herein, the term "inflammatory disease" may be defined as a pathological symptom, which is caused by an inflammatory response characterized as a local or systemic biological defense response to autoimmunity or infection from external physical and chemical stimuli or external infectious agents (e.g., bacteria, fungi, viruses, various allergens, etc.). The inflammatory response involves a series of complex physiological reactions, such as activation of various inflammatory mediators and enzymes associated with immune cells (e.g., iNOS, COX-2, etc.), secretion of inflammatory mediators (e.g., secretion of TNF-α, IL-6, IL-8, IL-17, IL-1β, IFN-γ, etc.), infiltration of body fluids, cell migration, tissue destruction, etc., and may appear externally through symptoms, such as erythema, pain, edema, fever, and deterioration or loss of certain functions of the body.

Since the inflammatory disease may be acute, chronic, ulcerative, allergic, or necrotic disease, it includes all acute, chronic, ulcerative, allergic, or necrotic diseases as as long as any disease is included as an inflammatory disease as described above.

Specifically, the inflammatory disease may be an inflammatory skin disease or inflammatory autoimmune disease.

The inflammatory skin disease may include atopic dermatitis, contact dermatitis, allergic skin disease, acne, and urticaria.

The inflammatory autoimmune disease may be psoriasis, systemic scleroderma, systemic lupus erythematosus, rheumatoid arthritis, asthma, ulcerative colitis, Behcet's disease, Crohn's disease, multiple sclerosis, dermatomyositis, collagen disease, vasculitis, arthritis, granulomatosis, organ-specific autoimmune lesion, graft-versus-host disease (GvHD), etc.

As used herein, the term "prevention" refers to all actions that inhibit or delay the occurrence, spread, and recurrence of inflammation by the peptide of the present invention or a composition including the same.

As used herein, the term "amelioration or treatment" refers to all actions that beneficially alter inflammation, such as delaying, stopping, or reversing the progression of inflammation by the peptide of the present invention or a composition including the same.

In a specific embodiment of the present invention, the peptide may prevent, ameliorate, or treat inflammatory diseases by inhibiting the expression of COX-2 and IFN-γ in splenocytes and the secretion of TNF-α, IL-17, IFN-γ, IL-1β, and IL-17.

In addition, the peptide may prevent, ameliorate, or treat inflammatory diseases by inhibiting the expression of inflammatory cytokines (e.g., TNF-α, IL-6, and IL-8) and COX2 and reducing the expression of inflammatory mediators (e.g., iNOS and JNK) in keratinocytes.

The pharmaceutical composition of the present invention may further include a pharmaceutically acceptable carrier.

As the pharmaceutically acceptable carrier, carriers for oral administration or carriers for parenteral administration may be further included. The carriers for oral administration may include lactose, starch, cellulose derivatives, magnesium stearate, stearic acid, etc. Additionally, the carriers for parenteral administration may also include water, suitable oils, salines, aqueous glucose and glycols, etc. Additionally, stabilizers and preservatives may be included. Suitable stabilizers include antioxidants, such as sodium bisulfite, sodium sulfite, or ascorbic acid. Suitable preservatives include benzalkonium chloride, methyl- or propyl-paraben, and chlorobutanol. As suitable pharmaceutically acceptable carriers, those described in Remington's Pharmaceutical Sciences (19th ed., Mack Publishing Company, Easton, PA, 1995) may be referred to.

The pharmaceutical composition of the present invention may be administered to mammals, including humans, by any method. For example, the composition may be administered orally or parenterally, and the parenteral administration method may be intravenous, intramuscular, intraarterial, intramedullary, intrathecal, intracardiac, transdermal, subcutaneous, intraperitoneal, intranasal, enteral, topical, sublingual, or rectal administration, but is not limited thereto.

The pharmaceutical composition of the present invention may be formulated into a preparation for oral or parenteral administration according to the administration route as described above, and the preparation for parenteral administration may specifically be used after formulating in the form of a preparation for injection or a preparation for external use.

The pharmaceutical composition of the present invention is administered in a pharmaceutically effective amount. In the present invention, the "pharmaceutically effective amount" refers to an amount sufficient to treat the disease with a reasonable benefit/risk ratio applicable to medical treatment, and the effective dose level may be determined based on factors including the type and severity of the patient's disease, activity of the drug, sensitivity to the drug, time of administration, route of administration and excretion rate, duration of treatment, and drugs used simultaneously, and other factors well known in the medical field. The pharmaceutical composition may be administered as an individual therapeutic agent or in combination with other anti-inflammatory agents, and may be administered simultaneously with, separately from, or sequentially with conventional therapeutic agents, and may be administered once or multiple times. Considering all of the above factors, it is important to administer an amount that can achieve the maximum effect with the minimum amount without side effects, and this can easily be determined by those skilled in the art.

The effective amount of the pharmaceutical composition may vary depending on the patient's age, sex, condition, body weight, degree of absorption of the active ingredient in the body, inactivation rate, excretion rate, type of disease, and drugs administered in combination, may increase or decrease depending on the route of administration, severity, sex, body weight, age, etc., for example, the pharmaceutical composition may be administered in an amount of about 0.0001 µg to about 500 mg, preferably 0.01 µg to 100 mg, per 1 kg of the patient's body weight per day.

Still another aspect of the present invention provides a cosmetic composition for anti-inflammation, which includes a peptide including the amino acid sequence of SEQ ID NO: 1 as an active ingredient.

The cosmetic composition may be prepared in any formulation commonly prepared in the art, for example, may be formulated into a solution, a suspension, an emulsion, a paste, a gel, a cream, a lotion, a powder, a soap, a surfactant-containing cleanser, an oil, a powder foundation, an emulsion foundation, a wax foundation, a spray, etc., but is not limited thereto.

The cosmetic composition includes a softening lotion, a nourishing lotion, a nourishing cream, a massage cream, an essence, an eye cream, a cleansing cream, a cleansing foam, cleansing water, a mask, a spray, a powder, a hair tonic, a hair cream, a hair lotion, a hair shampoo, a hair conditioner, a hair spray, a hair aerosol, a pomade, a solution (*e.g.*, gel, etc.), sol-gel, an emulsion, an oil, a wax, an aerosol, etc., but is not limited thereto.

The cosmetic composition of the present invention may include other additives such as excipients and carriers, and it is possible to apply and mix common ingredients being mixed in general skin cosmetics as necessary.

When the formulation of the cosmetic composition is a paste, cream, or gel, as carrier ingredients, animal oils, vegetable oils, wax, paraffin, starch, tragacant, cellulose derivatives, polyethylene glycol, silicone, bentonite, silica, talc, zinc oxide, *etc*. may be used.

When the formulation of the cosmetic composition is a powder or spray, as carrier ingredients, lactose, talc, silica, aluminum hydroxide, calcium silicate, or polyamide powder may be used; and especially in the case of a spray, additional propellants (*e.g.,* chlorofluorohydrocarbon, propane/butane, or dimethyl ether) may be included, but is not limited thereto.

When the formulation of the cosmetic composition is in the form of a solution or emulsion, as carrier ingredients, a solvent, a solubilizing agent or emulsifying agent may be used, for example, water, ethanol, isopropanol, ethyl carbonate, ethyl acetate, benzyl alcohol, benzyl benzoate, propylene glycol, 1,3-butyl glycol oil, glycerol aliphatic ester, a fatty acid ester of polyethylene glycol or sorbitan, etc. may be used.

When the formulation of the cosmetic composition is in the form of a suspension, as carrier ingredients, liquid diluents (*e.g.*, water, ethanol, propylene glycol, etc.), suspending agents (*e.g.*, ethoxylated isostearyl alcohol, polyoxyethylene sorbitol ester, polyoxyethylene sorbitan ester, *etc*.), microcrystalline cellulose, aluminum metahydroxide, bentonite, agar, tragacant, *etc*. may be used.

When the formulation of the cosmetic composition is a surfactant-containing cleanser, as carrier ingredients, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamidobetaine, aliphatic alcohol, fatty acid glyceride, fatty acid diethanolamide, vegetable oils, lanolin derivatives, ethoxylated glycerol fatty acid esters, etc. may be used.

When the formulation of the cosmetic composition is a hair shampoo, the peptide of the present invention may be mixed with base ingredients for formulating a shampoo, such as a thickener, a surfactant, a viscosity modifier, a humectant, a pH modifier, a preservative, an essential oil, etc. As the thickener, CDE may be used; as the surfactant, LES (*i.e.*, an anionic surfactant) and coco betaine (*i.e.*, an amphoteric surfactant) may be used; as the viscosity modifier, poly quarter may be used; as the humectant, glycerin may be used; and as the pH modifier, citric acid and sodium hydroxide may be used. As the preservative, a grapefruit extract, etc. may be used, and in addition, essential oils (*e.g.*, cedarwood, peppermint, and rosemary, *etc*.), silk amino acids, pentaol, or vitamin E may be added.

The ingredients to be included in the cosmetic composition may further include as active ingredients, in addition to the peptide and carrier ingredients of the present invention, adjuvants commonly used in cosmetic compositions, such as antioxidants, stabilizers, solubilizers, vitamins, pigments, and fragrances; etc., but are not limited thereto.

As described above, the peptide of the present invention has an excellent effect of inhibiting inflammation, and thus can be used as a raw material for pharmaceutical drugs for the purpose of preventing, ameliorating, or treating inflammatory diseases; or cosmetics for the purpose of ameliorating skin inflammation.

Still another aspect of the present invention provides a method for preventing or treating inflammatory diseases, which includes administering a therapeutically effective amount of a peptide including the amino acid sequence of SEQ ID NO: 1 to a subject.

The peptide including the amino acid sequence of SEQ ID NO: 1 is the same as the peptide described in the "a pharmaceutical composition for preventing or treating inflammatory diseases" section, and thus the specific description above is used.

As used herein, the term "therapeutically effective amount" refers to the amount of a peptide which is can achieve the goal of ameliorating the incidence of a disease during treatment with the peptide, but can avoid harmful side effects usually associated with other treatments.

Still another aspect of the present invention provides uses of the amino acid sequence of SEQ ID NO: 1 for use in the preparation of a drug for preventing or treating inflammatory diseases.

Hereinafter, the present invention will be described in detail through examples and experimental examples.

However, the following examples and experimental examples are only for illustrating the present invention, and the contents of the present invention are not limited by the following examples and experimental examples.

### MODE FOR CARRYING OUT THE INVENTION

### [Synthesis Example 1]

### Synthesis of Peptides Including Amino Acid Sequence of SEQ ID NO: 1

Peptides having the amino acid sequence of SEQ ID NO: 1 shown in [Table 1] below were synthesized using an automatic peptide synthesizer (Liberty, CEM Corporation, USA), and these synthesized peptides were purified using the C18 reverse phase high performance liquid chromatography (HPLC) (U-3000, Thermo Fisher Scientific, USA). The column used was Pursuit XRs C18 (250 mm × 4.65 mm 100Å, Agilent, USA).

**[Table 1]**

| SEQ ID NO | Amino Acid Sequence of Peptide |
|---|---|
| 1 | SVSVGMKPSPRP |

### [Experimental Example 1]

### Confirmation of effect of inhibiting LPS-induced inflammation in splenocytes

### <1-1> Confirmation of changes in expression of pro-inflammatory cytokine genes

In order to confirm the effect of the peptides of the present invention synthesized in Synthesis Example 1 on the inflammatory response induced by LPS, the expression levels of mRNA of inflammatory cytokines COX2 and INFγ were measured by RT-PCR.

Specifically, 6-7 week old mice (C57BL/6, Young Bio Co., Ltd.) were sacrificed to obtain spleens, which were crushed using a cell strainer, and then centrifuged along with serum-free RPMI-1640 medium. The supernatant was discarded and red blood cells (RBCs) were removed twice using RBC lysis buffer. Then, after washing the resultant with serum-free RPMI-1640 medium, an appropriate amount of serum-free RPMI-1640 medium was added. The number of mouse splenocytes was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and LPS (2 pg/mL), which is an inflammation-inducing substance, as a stimulant, and cultured for 3 additional hours.

Next, cells were recovered after culture, and total RNA was extracted using an RNA extraction kit (Qiagen RNeasy kit), and after RNA quantification, cDNA was synthesized using a cDNA synthesis kit (Intron, Korea). A polymerase chain reaction (PCR) was performed by mixing a PCR premix (Intron, Korea), synthesized cDNA, and primers shown in [Table 2] below specific for each of the genes COX2, INFγ, and GAPDH. PCR products were electrophoresed on a 5% agarose gel and stained with ethidium bromide to confirm the mRNA expression levels of COX2 and INFγ, genes associated with inflammation and autoimmune diseases.

**[Table 2]**

| Gene | Primer | Sequence (5'->3') | SEQ ID NO |
|---|---|---|---|
| COX2 | F | TGAGTGGTAGCCAGCAAAGC | 2 |
| | R | CTGCAGTCCAGGTTCAATGG | 3 |
| INFγ | F | GAGGTCAACAACCCACAGGT | 4 |
| | R | GGGACAATCTCTTCCCCACC | 5 |

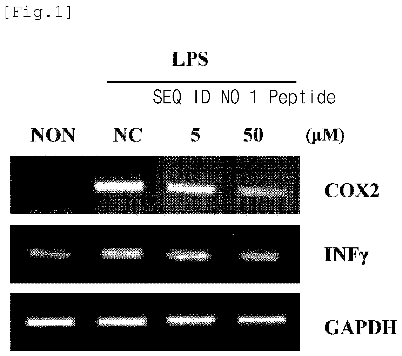

As a result, as shown in [FIG. 1], it was confirmed that treatment with the peptide of the present invention can significantly reduce the expression of inflammation-associated genes associated with even after creating an inflammatory environment with LPS in splenocytes.

### <1-2> Confirmation of amount of pro-inflammatory cytokines secreted

In order to confirm the effect of the peptides of the present invention synthesized in Synthesis Example 1 on the inflammatory response induced by LPS, the secretion amounts of inflammatory cytokines TNF-α, IL-17, and INFγ were measured by ELISA.

Specifically, 6-7 week old mice (C57BL/6, Young Bio Co., Ltd.) were sacrificed to obtain spleens, which were crushed using a cell strainer, and then centrifuged with serum-free RPMI-1640 medium. The supernatant was discarded and red blood cells (RBCs) were removed twice using RBC lysis buffer. Then, after washing the resultant with serum-free RPMI-1640 medium, an appropriate amount of serum-free RPMI-1640 medium was added thereto. The number of mouse splenocytes was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and LPS (2 pg/mL), which is an inflammation-inducing substance, as a stimulant, and cultured for 24 additional hours.

Next, the cell culture was recovered after cultivation, and the amounts of secretion of IL-17, IFN-γ, and TNF-α, which are secretion markers during Th17 differentiation, were measured using an ELISA kit (Quantikine ELISA Kit, R&D System, USA) specific to IL-17, IFN-γ, and TNF-α, and the absorbance was measured using Spectramax M2 (Molecular Devices).

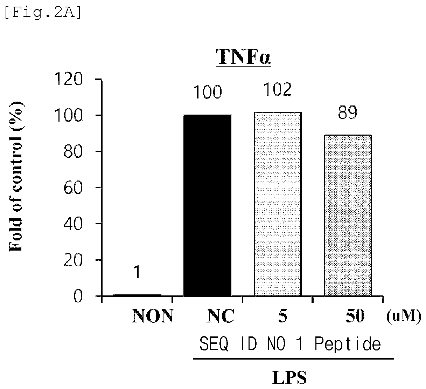

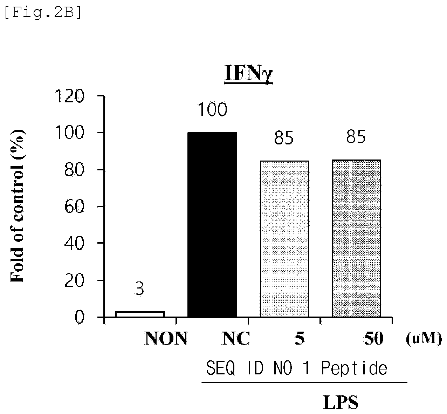

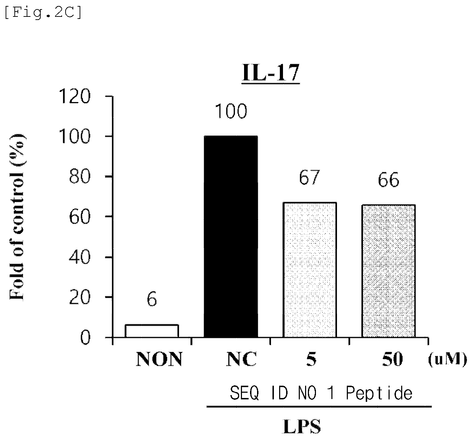

As a result, as shown in FIGS. 2A to 2C, it was confirmed that the peptide of the present invention can significantly reduce the secretion of TNF-α, IFN-γ, and IL-17, which are inflammatory cytokines.

### [Experimental Example 2]

### Confirmation of effect of inhibiting inflammation induced by various inflammation-inducing substances in splenocytes

### <2-1> Confirmation of effect of inhibiting inflammation induced by TNF-α

In order to confirm the effect of the peptides of the present invention synthesized in Synthesis Example 1 on the inflammatory response induced by TNF-α, the secretion amounts of TNF-α, IL-1β, and INFγ, which are inflammatory cytokines, were measured by ELISA.

Specifically, 6-7 week old mice (C57BL/6, Young Bio Co., Ltd.) were sacrificed to obtain spleens, which were crushed using a cell strainer, and then centrifuged with serum-free RPMI-1640 medium. The supernatant was discarded and red blood cells (RBCs) were removed twice using RBC lysis buffer. Then, after washing the resultant with serum-free RPMI-1640 medium, an appropriate amount of serum-free RPMI-1640 medium was added thereto. The number of mouse splenocytes was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and TNF-α (20 nM), which is an inflammation-inducing substance, as a stimulant, and cultured for 24 additional hours.

Next, the cell culture was recovered after cultivation, and the amounts of secretion of TNF-α, IL-1β, and INFγ, which are inflammatory cytokines, were measured using an ELISA kit (Quantikine ELISA Kit, R&D System, USA) specific to TNF-α, IL-1β, and INFγ, and the absorbance was measured using Spectramax M2 (Molecular Devices).

As a result, as shown in FIGS. 3A to 3C, it was confirmed that the peptide of the present invention can significantly reduce the secretion of TNF-α, IL-1β, and INFγ, which are inflammatory cytokines.

### <2-2> Confirmation of effect of inhibiting inflammation induced by LPS, TGF-β, and IL-23

In order to confirm the effect of the peptides of the present invention synthesized in Synthesis Example 1 on the inflammatory response induced by LPS, TGF-β, and IL-23, the secretion amount of INFγ, which is an inflammatory cytokine, was measured by ELISA.

Specifically, 6-7 week old mice (C57BL/6, Young Bio Co., Ltd.) were sacrificed to obtain spleens, which were crushed using a cell strainer, and then centrifuged with serum-free RPMI-1640 medium. The supernatant was discarded and red blood cells (RBCs) were removed twice using RBC lysis buffer. Then, after washing the resultant with serum-free RPMI-1640 medium, an appropriate amount of serum-free RPMI-1640 medium was added thereto. The number of mouse splenocytes was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and LPS (2 pg/mL), TGF-β (20 ng/mL), IL-23 (20 ng/mL), which are inflammation-inducing substances, as a stimulant, and cultured for 24 additional hours.

Next, the cell culture was recovered after cultivation, and the amount of secretion of INFγ, which is an inflammatory cytokine, was measured using an ELISA kit (Quantikine ELISA Kit, R&D System, USA) specific to INFγ, and the absorbance was measured using Spectramax M2 (Molecular Devices).

As a result, as shown in FIG. 4, it was confirmed that the peptide of the present invention can significantly reduce the secretion of INFγ, which is an inflammatory cytokine.

### <2-3> Confirmation of effect of inhibiting inflammation induced by TNF-α and IL-17A

In order to confirm the effect of the peptides of the present invention synthesized in Synthesis Example 1 on the inflammatory response induced by TNF-α and IL-17A, the secretion amount of IL-17, which is an inflammatory cytokine, was measured by ELISA.

Specifically, 6-7 week old mice were sacrificed to obtain spleens, which were crushed using a cell strainer, and then centrifuged with serum-free RPMI-1640 medium. The supernatant was discarded and red blood cells (RBCs) were removed twice using RBC lysis buffer. Then, after washing the resultant with serum-free RPMI-1640 medium, an appropriate amount of serum-free RPMI-1640 medium was added thereto. The number of mouse splenocytes was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and TNF-α (20 nM) and IL-17A (200 ng/mL), which are inflammation-inducing substances, as a stimulant, and cultured for 24 additional hours.

Next, the cell culture was recovered after cultivation, and the amount of secretion of IL-17, which is an inflammatory cytokine, was measured using an ELISA kit (Quantikine ELISA Kit, R&D System, USA) specific to INFγ, and the absorbance was measured using Spectramax M2 (Molecular Devices).

As a result, as shown in FIG. 5, it was confirmed that the peptide of the present invention can significantly reduce the secretion of IL-17, which is an inflammatory cytokine.

### [Experimental Example 3]

### Confirmation of effect of inhibiting inflammation induced by Th17 in keratinocytes

### <3-1> Confirmation of changes in expression of pro-inflammatory cytokine genes

In order to confirm the effect of the peptides of the present invention synthesized in Synthesis Example 1 on Th17-mediated inflammatory responses, the mRNA expression levels of TNF-α, COX2, IL-6, and IL-8 were measured by RT-PCR.

Specifically, the number of human keratinocytes HaCaT (CLS Cell Lines Service)) was measured, and the cells were inoculated into a 24-well plate at a density of 3 × 10⁵ cells/well, and cultured overnight. Next day, the medium was replaced with serum-free medium and the cells were cultured for 4 hours, and then treated with the peptide of the present invention (5 µM and 50 µM) and TNF-α (10 nM) and hIL-17A (200 ng/mL), which are Th17 type inflammation-inducing cytokines, used as a stimulant, and cultured for one additional hour.

Next, cells were recovered after culture, and total RNA was extracted using an RNA extraction kit (Qiagen RNeasy kit), and after RNA quantification, cDNA was synthesized using a cDNA synthesis kit (Intron, Korea). A polymerase chain reaction (PCR) was performed by mixing a PCR premix (Intron, Korea), synthesized cDNA, and primers shown in [Table 3] below specific for each of the genes of TNF-α, COX2, IL-6, IL-8, and GAPDH. PCR products were electrophoresed on a 5% agarose gel and stained with ethidium bromide to confirm the mRNA expression levels of TNF-α, COX2, IL-6, and IL-8, which are genes associated with inflammation and autoimmune diseases.

**[Table 3]**

| Gene | Primer | Sequence (5'->3') | SEQ ID NO |
|---|---|---|---|
| TNF-α | F | AACATCCAACCTTCCCAAACG | 6 |
| | R | GACCCTAAGCCCCCAATTCTC | 7 |
| COX2 | F | ATCATTCACCAGGCAAATTGC | 8 |
| | R | GGCTTCAGCATAAAGCGTTTG | 9 |

| | | | |
|---|---|---|---|
| IL-6 | F | AAAGAGGCACTGCCAGAAAA | 10 |
| | R | ATCTGAGGTGCCCATGCTAC | 11 |
| IL-8 | F | GAAGGTGCAGTTTTGCCAAG | 12 |
| | R | ACCCTCTGCACCCAGTTTTC | 13 |
| GAPDH | F | GGAGCCAAAAGGGTCATCAT | 14 |
| | R | GTGATGGCATGGACTGTGGT | 15 |

As a result, as shown in [FIG. 6], it was confirmed that the peptide of the present invention can significantly reduce the expression of genes associated with inflammation and autoimmunity.

### <3-2> Confirmation of changes in activity of inflammatory cytokines

In order to confirm the effect of the peptides of the present invention synthesized in Synthesis Example 1 on Th17-mediated inflammatory responses, the degree of phosphorylation of iNOS and pJNK, which are inflammation-inducing factors and signaling proteins, was measured by Western blot.

Specifically, the number of human keratinocytes HaCaT (CLS Cell Lines Service)) was measured, and the cells were inoculated into a 24-well plate at a density of 1 × 10⁷ cells/well, and cultured overnight. Next day, the medium was replaced with serum-free medium and the cells were cultured for 4 hours, and then treated with the peptide of the present invention (5 µM and 50 µM) and TNF-α (10 nM) and hIL-17A (200 ng/mL), which are Th17 type inflammation-inducing cytokines, as a stimulant, and cultured for 24 additional hours.

After cultivation, the cells were recovered to prepare a cell lysate, and a western blot was performed using antibodies against iNOS and pJNK (Cell Signaling Technology, USA) to confirm the patterns of protein expression.

As a result, as shown in [FIG. 7], it was confirmed that the peptide of the present invention can reduce the phosphorylation of iNOS and JNK, which are inflammation-inducing factors and signaling proteins, even in a Th17-mediated inflammation-inducing environment.

From the above, the present invention has been described in detail only with respect to the described embodiments, but it is obvious to those skilled in the art that various changes and modifications are possible within the technical scope of the present invention, and it is natural that such changes and modifications fall within the scope of the appended claims.

## Claims

1. A peptide comprising the amino acid sequence represented by SEQ ID NO: 1.

2. The peptide of claim 1, wherein the peptide has anti-inflammatory activity.

3. An anti-inflammatory composition comprising the peptide of claim 1 as an active ingredient.

4. The anti-inflammatory composition of claim 3, wherein the peptide inhibits, in the splenocytes where an inflammatory response is induced, i) the expression of cyclooxygenase 2 (COX-2) and interferon gamma (INF-γ) or ii) the secretion of any one inflammatory cytokine selected from the group consisting of tumor necrosis factor alpha (TNF-α), INF-γ, interleukin 17 (IL-17), and interleukin 1 beta (IL-1β).

5. The anti-inflammatory composition of claim 3, wherein the peptide inhibits, in the keratinocytes where an inflammatory response is induced, the expression of cyclooxygenase 2 (COX-2) and any one inflammatory cytokine selected from the group consisting of TNF-α, interleukin 6 (IL-6), and interleukin 8 (IL-8).

6. The anti-inflammatory composition of claim 3, wherein the peptide inhibits, in the keratinocytes where an inflammatory response is induced, the activity of any one selected from the group consisting of inducible nitric oxide synthase (iNOS) and cyclooxygenase 2 (COX-2).

7. A pharmaceutical composition for preventing or treating an inflammatory disease comprising the peptide of claim 1 as an active ingredient.

8. The pharmaceutical composition of claim 7, wherein the inflammatory disease is an inflammatory skin disease.

9. The pharmaceutical composition of claim 8, wherein the inflammatory disease is selected from the group consisting of atopic dermatitis, contact dermatitis, allergic skin disease, acne, and urticaria.

10. The pharmaceutical composition of claim 7, wherein the inflammatory disease is an inflammatory autoimmune disease.

11. The pharmaceutical composition of claim 10, wherein the inflammatory autoimmune disease is selected from the group consisting of psoriasis, systemic scleroderma, systemic lupus erythematosus, rheumatoid arthritis, asthma, ulcerative colitis, Behcet's disease, Crohn's disease, multiple sclerosis, dermatomyositis, collagen disease, vasculitis, arthritis, granulomatosis, organ-specific autoimmune lesion, and graft-versus-host disease (GvHD).

12. A cosmetic composition for anti-inflammation comprising the peptide of claim 1 as an active ingredient.
